# EUROPEAN PATENT APPLICATION

(11) **EP 4 186 976 A1**
(43) Date of publication of application: **31.05.2023**
(21) Application number: 21847417.9
(22) Date of filing: 14.07.2021
(51) Int. Cl.: C12N 15/82, C12N 9/22, C12N 15/10

(54) **N-GLYCOSYLATION MUTANT RICE, METHOD FOR PREPARING SAME, AND METHOD FOR PREPARING RICE FOR PROTEIN PRODUCTION BY USING SAME**

(30) Priority: 21.07.2020 KR 20200090189
(71) Applicant: Phytomab Co., Ltd., Seoul 03911 (KR)
(72) Inventor: KIM, Seong, Ryong, Seoul 08637 (KR); SHIN, Jun, Hye, Seoul 06081 (KR)
(74) Representative: AWA Sweden AB
(86) International application number: PCT/KR2021/009025
(87) International publication number: WO 2022/019560

(57) **Abstract**

Provided are N-glycosylation mutant rice (Oryza sativa), a method of producing the same, and a method of producing rice for protein production using the same, wherein mutant cell lines for a total of 8 genes participating in the plant-specific N-glycosylation process are obtained, and the possibility of producing a medical protein therefrom is confirmed. Accordingly, the N-glycosylation mutant rice can be effectively used for protein production.

## Description

### Technical Field

The present disclosure was made with the task identification number 1395062825 and the detailed task number PJ013659012020 under the support of the Rural Development Administration, and the research management specialized institution of the task is the Rural Development Administration, the name of the research project is "Next Generation Biogreen 21 Project", the name of the research project is "Development of human body-like glycoprotein producing rice cell line and production of medical protein", the supervising institution is Seogang University Industry-University Cooperation Foundation, and the research period is from March 1, 2018 to December 31, 2020.

This patent application claims priority to Korean Patent Application No. 10-2020-0090189 filed with the Korean Intellectual Property Office on July 21, 2020, and the disclosure of which is incorporated herein by reference.

The present disclosure relates to N-glycosylation mutant rice *(Oryza sativa),* a method of producing the same, and a method of producing rice for protein production using the same, and more particularly, to a technique for establishing a rice cell line capable of producing medical proteins by removing plant-specific glycosylation through the edition of a total of eight genes which participate in a plant-specific N-glycosylation process.

### Background Art

In all eukaryotic organisms including plants, post translational modification of proteins is an essential process for final protein activity. From among the post translation modification process, the N-glycosylation process is a modification process for membrane proteins or secretory proteins, in which a special sugar chain is attached to the asparagine residue in the motif of asparagine (ASN)-X (amino acid except proline)-serine or threonine (Ser/Thr) in the sequence of a protein. The polymerization process of these sugar chains affects the activity, structure, action site, substrate recognition, and stabililty of glycoproteins.

N-glycosylation process of proteins starting from the endoplasmic reticulum occurs commonly in all studied eukaryotes, in which oligomannosidic N-glycan form with mannose exposed is attached. Such sugar chains are converted into complex sugar chains in the Golgi body, and in this process, various sugars, such as fucose, xylose, and galactose, are attached. This process is called a complex N-glycosylation.

The complex N-glycosylation process slightly varies depending on an organism. In particular, unlike animals, in the case of plants, α1,3 fucose and β1,2 xylose are specifically attached, and in the final conversion process that takes place in the trans-Golgi, α1,4 fucose and β1,3 galactose are attached.

Most of the receptors that detect environmental stimuli or stress may exist in the form of glycoproteins. Therefore, studies related to glycosylation are required to understand the mechanism of action of such proteins, and furthermore, the mechanism of action of plants in response to stress.

Until now, functional studies of N-glycosylation genes in plants have been mainly studied using Arabidopsis. As a result, it was confirmed that mutants of genes participating in N-glycosylation in the endoplasmic reticulum underwent modification of cell wall structure and inhibition of root growth, and increase in sensitivity to stress.

In the case of Arabidopsis and tobacco, significant phenotypic changes were not observed in the mutants of the genes participating in the complex N-glycosylation, and there were no significant obstacles to survival.

In contrast, in the case of rice, which is a monocotyledonous model plant, mutations of the complex N-glycosylation genes showed various phenotypes at developmental stages, and in particular, sensitivity thereof was increased with respect to salt stress treatment.

These results suggest that the complex N-carbohydrate structure is not essential for vital phenomena or stress responses in Arabidopsis and tobacco, whereas the complex N-carbohydrate structure has a more sensitive effect on ER stress, etc. in rice (*Oryza sativa*). This implies that the complex N-carbohydrate structure of rice may perform an unknown important function. However, functional studies of these genes and stress-related studies with respect to rice are still in their initial stages. In addition, according to previous studies, mutations in rice N-glycosylation-related genes had a fatal effect on life, making it difficult to study the function of the gene.

Although the N-glycosylation process of proteins is known to have an important effect on the function or stability of proteins, there are little studies on N-glycosylation in plants. Most of the receptors that detect environmental stimuli or stress in plants and proteins that act on cell wall construction are expected to exist in the form of glycoproteins. Therefore, studies related to glycosylation are required to understand the mechanism of action of such proteins, and furthermore, the mechanism of action of plants in response to stress.

The biopharmaceutical market is rapidly growing all over the world, and in Korea, it is considered as one of the major next-generation businesses. Until now, biopharmaceuticals have been produced using animal cells, *Escherichia coli*, or yeast, but they have various long-lasting problems such as production costs. Therefore, due to these disadvantages, attempts are being made to produce medical proteins using plant systems.

However, since the N-glycosylation of mammals, including humans, is different from that of plants, the development of human-type N-glycosylated plant materials that overcome these problems is a very urgent issue.

### Detailed Description of the Invention

### Technical Problem

The inventors of the present application obtained mutant cell lines for a total of eight genes participating in the plant-specific N-glycosylation process in order to identify the function of the plant-specific N-glycosylation process on environmental stress in rice (*Oryza sativa*), and found that the mutant cell lines can be used as a plant system for the production of target proteins.

Accordingly, an objective of the present disclosure is to provide a vector for editing a rice N-glycosylation gene including a guide RNA consisting of one or more nucleotide sequences selected from the group consisting of SEQ ID NOS: 1 to 16.

An objective of the present disclosure is to provide an N-glycosylation mutant rice transformed with a vector including a guide RNA consisting of one or more nucleotide sequences selected from the group consisting of SEQ ID NOS: 1 to 16.

An objective of the present disclosure is to provide a method of producing an N-glycosylation mutant rice including transforming rice with a vector for editing rice N-glycosylation gene, the vector including a guide RNA consisting of one or more nucleotide sequences selected from the group consisting of SEQ ID NOS: 1 to 16.

An objective of the present disclosure is to provide a composition for producing trastuzumab (TMab), which is codon-optimized so that TMab is expressed in rice and includes a TMab light chain synthetic gene consisting of the nucleotide sequence of SEQ ID NO: 44 and a TMab heavy chain synthetic gene consisting of the nucleotide sequence of SEQ ID NO: 45.

An objective of the present disclosure is to provide a vector for expressing TMab, which is codon-optimized so that TMab is expressed in rice and includes a TMab light chain synthetic gene consisting of the nucleotide sequence of SEQ ID NO: 44 and a TMab heavy chain synthetic gene consisting of the nucleotide sequence of SEQ ID NO: 45.

An objective of the present disclosure is to provide a rice for producing TMab, the rice transformed with: a vector for editing a rice N-glycosylation gene including a guide RNA consisting of one or more nucleotide sequences selected from the group consisting of SEQ ID NOS: 1 to 16; and
a vector for expressing TMab, which is codon-optimized so that TMab is expressed in rice and includes a TMab light chain synthetic gene consisting of the nucleotide sequence of SEQ ID NO: 44 and a TMab heavy chain synthetic gene consisting of the nucleotide sequence of SEQ ID NO: 45.

An objective of the present disclosure is to provide a method of producing rice for protein production, the method including:
a gene editing step of transforming rice with a vector for editing a rice N-glycosylation gene including a guide RNA consisting of one or more nucleotide sequences selected from the group consisting of SEQ ID NOS: 1 to 16; and
a gene introduction step of transforming rice with a vector containing a gene encoding a target protein.

An objective of the present disclosure is to provide a protein production use of plant-specific N-glycosylation mutant rice.

### Technical Solution to Problem

The present disclosure relates to N-glycosylation mutant rice (*Oryza sativa*), a method of producing the same, and a method of producing rice for protein production using the same. The N-glycosylation mutant rice according to the present disclosure can be used as a plant system for production of a target protein.

The inventors of the present application studied the effects of functional defects of genes participating in the plant-specific glycosylation process on life phenomena at the plant cell level by using mutant cell lines of the genes, to help the understanding of N-glycosylation in plants.

To identify how the plant-specific N-glycosylation process affects environmental stress in rice, a total of 8 genes participating in the process were mutated using CRISPR-Cas9 technology, and as a result, functional mutant cell lines of 8 genes (β1,2-XyIT, α1,3-FucT, β1,3-GalT, α1,4-FucT, HEXO1, HEXO2, HEXO3, and HEXO4) and functional mutant cell lines of 4 genes (α1,3-FucT, β1,2-XylT, β1,3-GalT, α1,4-FucT) were obtained, and found that they could be used as a plant system for producing target proteins.

By using the cell line system from which plant-specific glycosylation is removed, the stress mechanism is effectively performed at the cellular level, and a protein production system is established, for use as a platform for the production of useful proteins such as protein drugs.

Hereinafter, the present disclosure will be described in more detail.

One aspect of the present disclosure is a guide RNA consisting of one or more nucleotide sequences selected from the group consisting of SEQ ID NOS: 1 to 16.

In an embodiment, the guide RNA may target one or more genes selected from the group consisting of β1,2-XylT, α1,3-FucT, β1,3-GalT, α1,4-FucT, HEXO1, HEXO2, HEXO3, and HEXO4.

These genes correspond to genes that function in plant-specific glycosylation processes.

In an embodiment, the guide RNA may be a polycistronic guide RNA designed to express two or more nucleotide sequences selected from the group consisting of SEQ ID NOS: 1 to 16.

The term "polycistronic" used herein refers to a state in which a plurality of amino acid sequences (cistrons), which are defined according to translation initiation and translation termination signals, exist in mRNA derived from one transcription unit. On the other hand, when only one is present, it is called a monocistron.

In an embodiment of the present disclosure, polycistronic guide RNAs designed to express eight nucleotide sequences selected from the group consisting of SEQ ID NOS: 1 to 16 were synthesized and used. The eight nucleotide sequences may be selected from the group consisting of SEQ ID NOS: 1, 3, 5, 7, 9, 11, 13, and 15, or may be selected from the group consisting of SEQ ID NOS: 1, 2, 3, 4, 5, 6, 7, and 8, and are not limited thereto.

An aspect of the present disclosure is to provide a vector for editing a rice N-glycosylation gene including a guide RNA consisting of one or more nucleotide sequences selected from the group consisting of SEQ ID NOS: 1 to 16.

The term "vector" refers to a means for expressing a target gene in a host cell. The vector refers to DNA fragment(s), nucleic acid molecules that are delivered into a cell, can replicate DNA, and reproduce independently in a host cell.

"Expression vector" refers to a recombinant DNA molecule containing a target coding sequence and an appropriate nucleic acid sequence that are essential for expressing an operably linked coding sequence in a particular host organism. The expression vector may include one or more of origins of replication, promoters, selectable markers, enhancers, termination signals, and polyadenylation sequences, which are all usable in eukaryotic cells. Expression vectors may be generally derived from plasmid or viral DNA, or may include the element of both. Thus, the expression vector refers to a recombinant DNA or RNA construct, such as plasmid, phage, recombinant virus or another vector that derives the expression of cloned DNA when being introduced into a suitable host cell. Suitable expression vectors are well known to one of ordinary skilled in the art, and may include those that are replicable in eukaryotic cells and/or prokaryotic cells and those that remain as episomal, or those that are integrated into the host cell genome.

Known vectors include pROKII, pBI76, pET21, pSK(+), pLSAGPT, pUC, and pGEM. Although not limited thereto, the recombinant vector may be a recombinant plant expression vector.

An example of a plant expression vector is a Ti-plasmid vector which, when present in a suitable host such as Agrobacterium tumefaciens, is capable of transferring a part of itself, the so-called T-region, into plant cells. Another type of Ti-plasmid vector (see EP 0 116 718 B1) is currently used to transfer hybrid DNA sequences into plant cells, or into protoplasts which is properly integrated into the genome of the plant and from which new plants can be produced. An example of the Ti-plasmid vector is a binary vector as claimed in EP 0 120 516 B1 and US patent No. 4,940,838.

Other suitable vectors according to the present disclosure that can be used to introduce DNA into plant hosts include viral vectors, such as those that can be derived from double-stranded plant viruses (for example, CaMV) and single-stranded viruses, gemini viruses, and the like, and may be selected from incomplete plant viral vectors. The use of such vectors can be particularly advantageous for the cases where it is difficult to properly transform a plant host. Expression vectors may include one or more selectable markers. The markers may be a nucleic acid sequence having a characteristic that can be selected by a conventional chemical method, and includes all genes capable of distinguishing transformed cells from non-transformed cells. Examples thereof are herbicide resistance genes, such as glyphosate or phosphinotricin, and antibiotic resistance genes, such as kanamycin, G418, bleomycin, hygromycin, and chloramphenicol, but are not limited thereto.

In an embodiment of the present disclosure, the promoter of the plant expression vector may be CaMV 35S, actin, ubiquitin, pEMU, MAS, or histone promoters, but is not limited thereto. The term "promoter" used herein refers to a region of DNA upstream from a structural gene and refers to a DNA molecule to which RNA polymerase binds to initiate transcription. A "plant promoter" is a promoter capable of initiating transcription in a plant cell. A "constitutive promoter" is a promoter that is active under most environmental conditions and states of development or cell differentiation. Since selection of transformants may be made by various tissues at various stages, constitutive promoters may be used. Thus, constitutive promoters do not limit selection possibilities. In addition, the vector may use a terminator of the related art, for example, nopaline synthase (NOS), a rice α-amylase RAmy1 A terminator, a phaseoline terminator, a terminator of octopine gene of agrobacterium tumefaciens, etc., but is not limited thereto. Regarding the need for terminators, it is generally known that such regions increase the certainty and efficiency of transcription in plant cells. Therefore, terminators may be used herein.

The recombinant vector may be constructed using a prokaryotic or eukaryotic cell as a host. For example, when the vector used is an expression vector and a prokaryotic cell is used as a host, a strong promoter capable of promoting transcription (e.g., pLλ promoter, CMV promoter, trp promoter, lac promoter, tac promoter, T7 promoter, etc.), a ribosome binding site for initiation of translation, and a transcription/translation termination sequence may be included. In the case of using a eukaryotic cell as a host, the origin of replication operating in the eukaryotic cell included in the vector may include a f1 origin of replication, a SV40 origin of replication, a pMB1 origin of replication, an adeno origin of replication, an AAV origin of replication, and a BBV origin of replication, and is not limited thereto. In an embodiment, a polyadenylation sequence may be included as a transcription termination sequence.

Plant transformation refers to any method of transferring DNA into a plant. Such transformation methods need not necessarily have periods of regeneration and/or tissue culture. Transformation of plant species is now common for plant species including monocotyledonous plants and dicotyledonous plants. In principle, any transformation method can be used to introduce the hybrid DNA according to the present disclosure into suitable progenitor cells. The method can be suitably selected from the calcium/polyethylene glycol method for protoplasts (Krens, FA et al., 1982, Nature 296, 72-74; Negrutiu I. et al., 1987, Plant Mol. Biol. 8, 363-373), electroporation of protoplasts (Shillito RD et al., 1985 Bio/Technol. 3, 1099-1102), microinjection into plant elements (Crossway A. et al., 1986, Mol. Gen. Genet. 202, 179-185), particle bombardment of various plant elements (DNA or RNA-coated) (Klein TM et al., 1987, Nature 327, 70), (incomplete) infection by viruses in Agrobacterium tumefaciens mediated gene transfer by infiltration of plants or transformation of mature pollen or microspores (EP 0 301 316), etc. In an embodiment of the present disclosure, the method may include Agrobacterium mediated DNA transfer.

A "plant cell" used for plant transformation may be any type of cultured cell, cultured tissue, cultured organ or whole plant. In an embodiment, the plant cell may be a cultured cell, a cultured tissue, or a cultured organ. In an embodiment, the plant cell may be any type of cultured cell.

"Plant" refers to differentiated or undifferentiated plant tissues such as, but not limited to, roots, stems, leaves, pollen, seeds, cancer tissues, and various types of cells used in culture, i.e., single cells, protoplasts, buds, and callus tissues. Plant tissues may be in planta or may be in organ culture, tissue culture or cell culture.

The method of selecting the transformed host cell can be easily performed according to a method widely known in the art using a phenotype expressed by a selection marker. For example, when the selection marker is a specific antibiotic resistance gene, the transformant may be easily selected by culturing the transformant in a medium containing the antibiotic.

In an embodiment, the guide RNA may target one or more genes selected from the group consisting of β1,2-XylT, α1,3-FucT, β1,3-GalT, α1,4-FucT, HEXO1, HEXO2, HEXO3, and HEXO4.

In an embodiment, the guide RNA may be a polycistronic guide RNA designed to express two or more nucleotide sequences selected from the group consisting of SEQ ID NOS: 1 to 16.

Another aspect of the present disclosure is to provide an N-glycosylation mutant rice transformed with a vector including a guide RNA consisting of one or more nucleotide sequences selected from the group consisting of SEQ ID NOS: 1 to 16.

In an embodiment, the guide RNA may target one or more genes selected from the group consisting of β1,2-XylT, α1,3-FucT, β1,3-GalT, α1,4-FucT, HEXO1, HEXO2, HEXO3, and HEXO4.

According to the present disclosure, the guide RNA may be a polycistronic guide RNA designed to express two or more nucleotide sequences selected from the group consisting of SEQ ID NOS: 1 to 16.

In an embodiment of the present disclosure, rice may be selected from the group consisting of cells, callus, seeds, and adults, and may be, for example, cells, but is not limited thereto.

Another aspect of the present disclosure is to provide a method of producing an N-glycosylation mutant rice including transforming rice with a vector for editing rice N-glycosylation gene, the vector including a guide RNA consisting of one or more nucleotide sequences selected from the group consisting of SEQ ID NOS: 1 to 16.

In an embodiment, the guide RNA may target one or more genes selected from the group consisting of β1,2-XylT, α1,3-FucT, β1,3-GalT, α1,4-FucT, HEXO1, HEXO2, HEXO3, and HEXO4.

According to the present disclosure, the guide RNA may be a polycistronic guide RNA designed to express two or more nucleotide sequences selected from the group consisting of SEQ ID NOS: 1 to 16.

In an embodiment of the present disclosure, rice may be selected from the group consisting of cells, callus, seeds, and adults, and may be, for example, cells, but is not limited thereto.

Another aspect of the present disclosure is to provide a composition for producing Trastzumab (TMab), which is codon-optimized so that TMab is expressed in rice and includes a TMab light chain synthetic gene consisting of the nucleotide sequence of SEQ ID NO: 44 and a TMab heavy chain synthetic gene consisting of the nucleotide sequence of SEQ ID NO: 45.

The term "codon optimization" in the present specification refers to a case in which the use of codons is optimized for each species in order to optimize protein synthesis. Since optimized codons vary depending on species, codon optimization is necessary even when a desired protein is to be efficiently expressed in other species. For example, when a human protein is expressed in E. coli, the protein can be expressed more efficiently when a codon is changed to a codon mainly used by E. coli from among the codons encoding the same amino acid.

Another aspect of the present disclosure is to provide a vector for expressing TMab, the vector which is codon-optimized so that TMab is expressed in rice and which includes a TMab light chain synthetic gene consisting of the nucleotide sequence of SEQ ID NO: 44 and a TMab heavy chain synthetic gene consisting of the nucleotide sequence of SEQ ID NO: 45.

Another aspect of the present disclosure is to provide a rice for producing TMab transformed with: a vector for editing a rice N-glycosylation gene including a guide RNA consisting of one or more nucleotide sequences selected from the group consisting of SEQ ID NOS: 1 to 16; and

a vector for expressing TMab, the vector which is codon-optimized so that TMab is expressed in rice and includes a TMab light chain synthetic gene consisting of the nucleotide sequence of SEQ ID NO: 44 and a TMab heavy chain synthetic gene consisting of the nucleotide sequence of SEQ ID NO: 45.

In an embodiment, the guide RNA may target one or more genes selected from the group consisting of β1,2-XylT, α1,3-FucT, β1,3-GalT, α1,4-FucT, HEXO1, HEXO2, HEXO3, and HEXO4.

According to the present disclosure, the guide RNA may be a polycistronic guide RNA designed to express two or more nucleotide sequences selected from the group consisting of SEQ ID NOS: 1 to 16.

In an embodiment of the present disclosure, rice may be selected from the group consisting of cells, callus, seeds, and adults, and may be, for example, cells, but is not limited thereto.

Another aspect of the present disclosure is to provide a method of producing rice for protein production, the method including:
a gene editing step of transforming rice with a vector for editing a rice N-glycosylation gene including a guide RNA consisting of one or more nucleotide sequences selected from the group consisting of SEQ ID NOS: 1 to 16; and
a gene introduction step of transforming rice with a vector containing a gene encoding a target protein.

In an embodiment, the target protein may be TMab.

In an embodiment, the guide RNA may target one or more genes selected from the group consisting of β1,2-XylT, α1,3-FucT, β1,3-GalT, α1,4-FucT, HEXO1, HEXO2, HEXO3, and HEXO4.

According to the present disclosure, the guide RNA may be a polycistronic guide RNA designed to express two or more nucleotide sequences selected from the group consisting of SEQ ID NOS: 1 to 16.

In an embodiment of the present disclosure, rice may be selected from the group consisting of cells, callus, seeds, and adults, and may be, for example, cells, but is not limited thereto.

### Advantageous Effects of Disclosure

The present disclosure relates to N-glycosylation mutant rice *(Oryza sativa),* a method of producing the same, and a method of producing rice for protein production using the same. In the present invention, mutant cell lines for a total of eight genes participating in the plant-specific N-glycosylation process were obtained, and it is confirmed that medical proteins can be produced from these mutant cell lines. Thus, the N-glycosylation mutant rice can be effectively used for protein production.

### Brief Description of Drawings

FIG. 1 shows images showing the result of an *in vitro* cleavage experiment to confirm the activity of sgRNA (single guide RNA) and Cas9.
FIG. 2A shows a schematic diagram of a pSK437 vector, which is a CRISPR-Cas9 vector.
FIG. 2B shows a schematic diagram of a pSK438 vector, which is a CRISPR-Cas9 vector.
FIG. 3 shows an image of a single cell (left) or a single cell-derived cell group (right) obtained from the suspension culture of #1-12-20-11 callus line.
FIG. 4 shows an image showing the result of immunoblotting for α1,3-Fucose and β of the obtained single cell line.
FIG. 5 shows an image of Trastzumab (TMab) expressed in a plant-specific glycosylation gene mutation single cell line.

### Best Mode

The present disclosure relates to an N-glycosylation mutant rice (*Oryza sativa*) transformed with a vector including a guide RNA consisting of one or more nucleotide sequences selected from the group consisting of SEQ ID NOS: 1 to 16.

### Mode of Disclosure

Hereinafter, the present disclosure will be described in more detail by the following examples. However, these examples are only for illustrating the present disclosure, and the scope of the present disclosure is not limited by these examples.

Throughout this specification, "%" used to indicate the concentration of a particular substance is (weight/weight)% for solids/solids, (weight/volume)% for solids/liquids, and (volume/volume)% for liquid/liquid.

### Example 1: Target sequence design and CRISPR-Cas9 vector construction

To prepare mutant rice (*Oryza sativa*) cell lines with respect to eight genes (β1,2-XylT, α1,3-FucT, β1,3-GalT, α1,4-FucT, HEXO1, HEXO2, HEXO3, HEXO4) and four genes (β1,2-XylT, α1,3-FucT, β1,3-GalT, α1,4-FucT) participating in plant-specific N-glycosylation and N-sugar modification processes, two optimal target sequences per gene were selected at the exon region of each gene by using CRISPR-P gRNA design tool (Liu et al., (2017) CRISPR-P 2.0: an improved CRISPR/Cas9 tool for genome editing in plants. Mol Plant 10: 530-532), and used for construction of a CRISPR-Cas9 vector.

Whether the sgRNA (single guide RNA)-Cas9 complex, selected by amplifying the target regions of the eight genes by PCR, can actually cleave DNA, was confirmed through an *in vitro* cleavage experiment. For these experiments, Cas9 was synthesized and purified by IPTG induction using *Escherichia coli* containing the Cas9 expression vector, pET28b-Cas9-His (#47327, addgene, www.addgene.org/47327/). Each of the *in vitro* transcribed gRNAs and the purified Cas9 protein were mixed with PCR products containing the target sequences of each gene and incubated at 37 °C for 2 hours. After the incubation, the reaction was terminated by treatment with an enzyme (proteinase K), and then DNA cleavage was confirmed by electrophoresis of a part of the reaction solution on a 1% agarose gel.

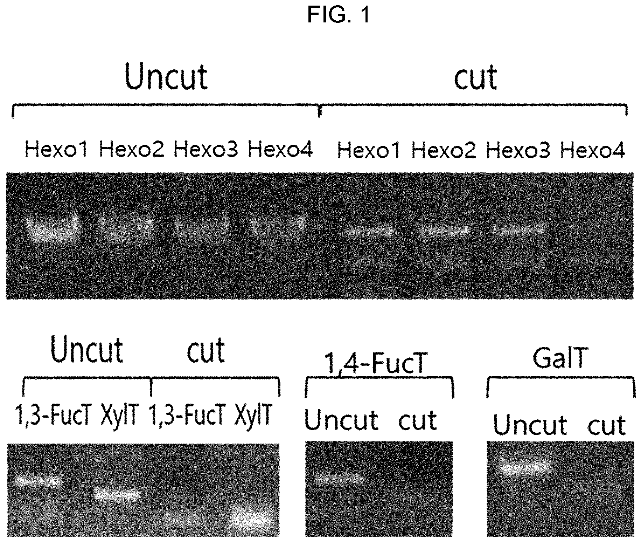

As can be seen in FIG. 1, it was confirmed that the sgRNA-Cas9 complex showed activity for each target.

For vector construction for 8 genes, one target guide RNA sequence was selected per gene corresponding to T1 in Table 1 below, and for vector construction for 4 genes, selected RNA sequences corresponding to T1 and T2 were all used.

**[Table 1]**

| | Target sequences of 8 genes that function in N-glycosylation | | | | |
|---|---|---|---|---|---|
| Sequence number | | Gene name | Guide | Sequence | Area |
| 1 | | α1,3-FucT | T1 | AGAGAGTATCCTCAGATCGA | Exon 2 |
| 2 | | | T2 | GGCCTTTGAGAATTCCAACG | Exon 4 |
| 3 | | β1,2-XylT | T1 | ACTCCTGTGAGGGGTACTTC | Exon 1 |
| 4 | | | T2 | GTTAGGTAAATCCGTGACTC | Exon 2 |
| 5 | | α1,4-FucT | T1 | GTACGGCGCCAACTCGACCG | Exon 1 |
| 6 | | | T2 | CGACCTTCCAAAGTTACCCA | Exon3 (or Exon 4) |
| 7 | | β1,3-GalT | T1 | TCATTCTTCGAATGGAATAT | Exon 1 |
| 8 | | | T2 | ATAAAAGTATCTCATCCACA | Exon 6 (or Exon 7) |
| 9 | | Hexo1 | T1 | GCTGCCGAGGAACTTCACCT | Exon 1 |
| 10 | | | T2 | TGCCTGGCCATGCAGAATCA | Exon 7 |
| 11 | | Hexo2 | T1 | GACCGGGTAGAAATTCCTGG | Exon 1 |
| 12 | | | T2 | GGGTGGAGCGAGCACCAGAG | Exon 2 |
| 13 | | Hexo3 | T1 | CTTGAAGGATGCCTTCCAGA | Exon 2 |
| 14 | | | T2 | ATGCCAACGTCGGTGAGCCA | Exon 1 |
| 15 | | Hexo4 | T1 | AGGGGAGCGTCGTCGAGGTG | Exon 1 |
| 16 | | | T2 | TTACTCAGAGAGATATACAA | Exon 7 |

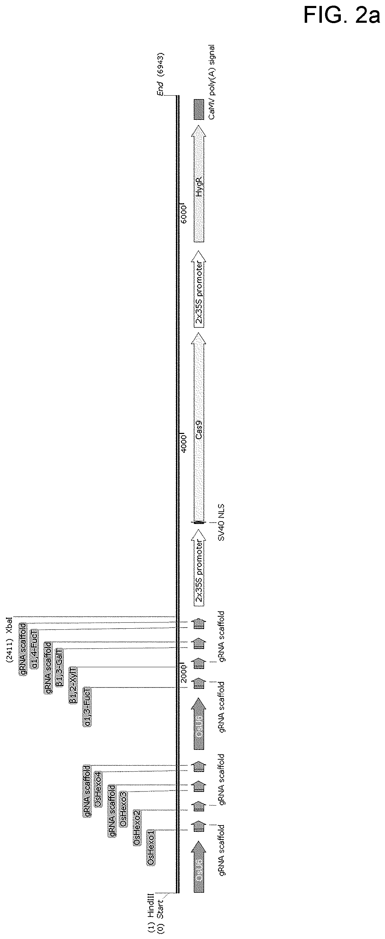

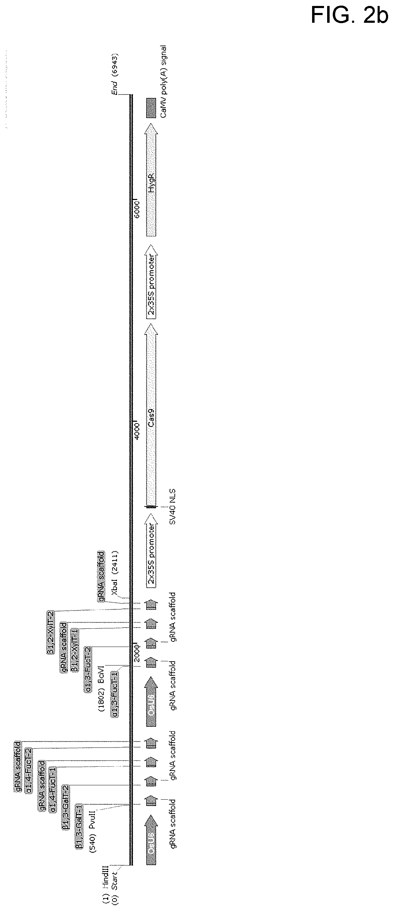

As can be seen in FIGS. 2A and 2A, each of polycistronic gRNA sequences designed to express target guide RNA sequences of eight genes (SEQ ID NOS: 1, 3, 5, 7, 9, 11, 13, and 15) and gRNA sequences of four genes (SEQ ID NOS: 1, 2, 3, 4, 5, 6, 7 and 8) was synthesized into the rice U6 promoter (GenScript, USA), and then pSK429 was constructed in which the herbicide resistance PPT gene, which is a selection marker of the pSB11-Cas9 vector (Shim et al., 2018), was substituted with the hygromycin resistance gene (HPT). pSK429 was cleaved with Hindlll and Xbal and the synthesized polystronic gRNA sequence was inserted, thereby preparing vectors pSK437 and pSK438. For plant transformation, *Agrobacterium tumefaciencs* (LBA4404) was transformed with the cloned CRISPR-Cas9 vectors.

### Example 2: Plant transformation and selection

Surface-sterilized rice (*Oryza sativa*) seeds were planted in 2N6 medium (hereinafter referred to as callus induction medium) supplemented with 2N6 salt (Duchefa, Haarlem, Netherlands) and 2 mg/L 2,4-D to induce the formation of callus for 3-4 weeks. Transformation of rice was performed by modifying the method of Hiei et al. (1994). Briefly, the formed callus was co-cultured in a suspension (OD₆₀₀ = 0.3) of Agrobacterium transformed with pSK437 and pSK438 at 23 °C for 3 days, and after 3 days, the callus was transferred to 2N6CH medium (hereinafter called callus selection medium) prepared by adding 40 mg/L hygromycin (hygromycin) and 200 mg/L cefotaxime to the callus induction medium. Thereafter, the calli were subcultured to a new medium every two weeks, and the transformed callus was finally selected therefrom.

### Example 3: Selection of transgenic callus

After selecting hygromycin-resistant callus in 2N6CH medium, transgenicity of the callus was identified by PCR using primer sequences for neighboring sequences including the target sequence as shown in Table 2.

**[Table 2]**

| | Primers used for PCR | | | |
|---|---|---|---|---|
| SEQ ID NO | | Name | Sequence (5' to 3') | Size |
| 17 | | α1,3-FucT-F_T1 | CCCTCAAGCTTTATGCTCAACT | 803 bp |
| 18 | | α1,3-FucT-R_T1 | GTTCCGTAGCTGGGGATACAT | |
| 19 | | α1,3-FucT-F_T2 | TTGTTTTGGACATTGATGCAC | 456 bp |
| 20 | | α1,3-FucT-R_T2 | TTCCACCCCAGAGAGATGAC | |
| 21 | | β1,2-XylT-F_T1 | CACCACAACAACAGCAACAAC | 531 bp |
| 22 | | β1,2-XylT-R_T1 | GTACTTGGGCAGCTCCTCCT | |
| 23 | | β1,2-XylT-F_T2 | CCTATTGTTATATTGCAGGATTCA | 458 bp |
| 24 | | β1,2-XylT-R_T2 | GAATCACATTATAAAGGAGCGAAGG | |
| 25 | | β1,3-Gal-F_T1 | TGCAGTTCAGAATCCACAGAA | 469 bp |
| 26 | | β1,3-Gal-R_T1 | AAGCACAATTGGAGGGTCTG | |
| 27 | | β1,3-Gal-F_T2 | CCATCTGCATCTATGGGGTA | 323 bp |
| 28 | | β1,3-Gal-R_T2 | TGATGGTGCACACAATAACTTAAA | |
| 29 | | α1,4-FucT-F_T1 | CTCCCACCCTTTCCACTGTA | 691 bp |
| 30 | | α1,4-FucT-R_T1 | ACGTGTACACCCCGTCGAG | |
| 31 | | α1,4-FucT-F_T2 | TCTTCACTGCGCAATGTCAC | 482 bp |
| 32 | | α1,4-FucT-R_T2 | GCCACAAAAATATCACCTCGT | |
| 33 | | Hexo1-F_T1 | ATACCCGGGCACATTTACAG | 483 bp |
| 34 | | Hexo1-R_T1 | CCACTCCAAGCTCCAGCTAC | |
| 35 | | Hexo2-F_T1 | CGACGAGTCCTACACGCTCT | 369 bp |
| 36 | | Hexo2-R_T1 | GAGTAGGAGCCGGAGTTGG | |
| 37 | | Hexo3-F_T1 | CATTACGAAATGGCTTTTCCAT | 535 bp |
| 38 | | Hexo3-R_T1 | TGCTATTCAACAGGCCAAGTTA | |
| 39 | | Hexo4-F_T1 | GGCGTTTCTCTTCATCTTCTTG | 559 bp |
| 40 | | Hexo4-R_T1 | CAGCTCTATCAGCGTCACCA | |

The PCR product was purified using a kit (Expin PCR SV mini kit, GeneAll, Korea), followed by Sanger sequencing. The obtained sequence was analyzed.

INDEL analysis of nucleotide sequences near the target sequence was performed using the analysis tool [Inference of CRISPR Edits (ICE) analysis tool (ice.synthego.com/#/)], and calli with the highest editing efficiency were selected therefrom.

As a result, in obtaining mutant cell lines of 8 genes from the group using the pSK437 vector, a rice callus line #1-12-20-11 exhibiting nearly 100% gene editing efficiency in 6 genes except for β1,3-GalT and α1,4-FucT genes was selected.

Selecting for obtaining mutant cell lines of the four genes from the group using the pSK438 vector was performed in the same manner as described above.

### Example 4: Obtaining gene-edited single cell line using suspension culture

The callus of line #1-12-20-11, which showed the highest editing efficiency, was inoculated into 2N6 liquid medium, cultured in suspension at 28 °C and 110 rpm, and subcultured every 2 weeks to obtain a single cell line.

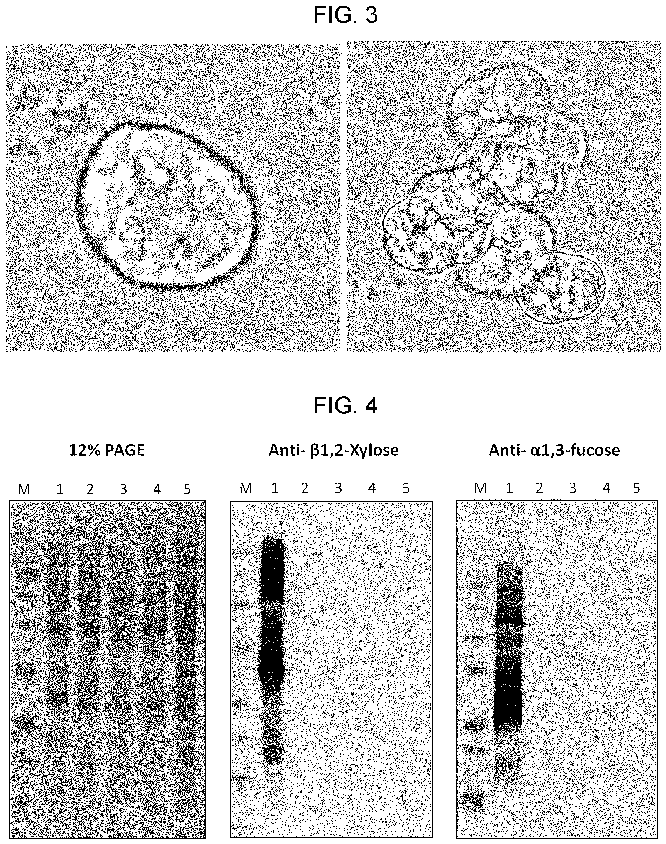

The suspension culture was filtered using a sieve (100 µm pore size), and single cells or single cell-derived cell groups obtained therefrom are shown in FIG. 3.

The cells collected by centrifugation were resuspended in 1 ml of 2N6 liquid medium, then spread on 2N6CH agar medium to grow until they reached a visible size, and each cell line grown on 2N6CH agar medium was proliferated and then, separated and grown on a new 2N6CH agar medium. In order to finally obtain a cell line derived from a single cell, the callus was removed from two different sites for each grown cell line callus, and genomic DNA was extracted therefrom, followed by PCR on the nucleotide sequences near target sequence.

INDEL analysis was performed using the nucleotide sequences of PCR products from two different sites of one cell line, and cell lines showing the same INDEL pattern were finally evaluated as single-cell-derived cell lines (#SC). As a result, two cell lines were obtained from #SC-26, an eight gene mutant cell line, and were named PMOsC1 and PMOsC2, respectively. In order to obtain four gene mutant cell lines, two single cell-derived cell lines were finally selected using #19 callus in the same manner as described above, and were named PMOsC3 and PMOsC4, respectively. The gene editing efficiency for each cell line is summarized in Table 3.

**[Table 3]**

| | Efficiency of gene editing for genes in the final selected cell line | | | | | | | | |
|---|---|---|---|---|---|---|---|---|---|
| Line number | | β1,2-XylT | α1,3-FucT | β1,3-GalT | α1,4-FucT | Hexo1 | Hexo2 | Hexo3 | Hexo4 |
| PMOsC1 | | 96 | 97 | 66 | 7 | 98 | 95 | 99 | 100 |
| PMOsC2 | | 95 | 98 | 78 | 16 | 97 | 91 | 98 | 100 |
| PMOsC3 | | 97 | 95 | 97 | 100 | - | - | - | - |
| PMOsC4 | | 97 | 95 | 97 | 100 | - | - | - | - |

### Example 5: Analysis of N-carbohydrate structure of obtained cell line

Immunoblotting and liquid chromatography-mass spectrometry (LC-MS) were used to identify the N-carbohydrate structure of the selected cell lines.

For immunoblotting, first, total protein was extracted from the cell line, and the relative amounts of α1,3-Fucose and β1,2-Xylose, which are plant-specific N-hydrocarbonates, were compared with wild-type (WT) Dongjin rice by western blotting. Protein was extracted from a PBS buffer solution (pH 7.4), and after 10 µg of total protein was subjected to PAGE electrophoresis, immunoblotting was performed thereon using anti-α1,3-Fucose (Agrisera, Sweden) and anti-β1,2-Xylose (Agrisera, Sweden) (M, size marker; Lane 1, Dongjin rice (WT); lane 2, PMOsC1; lane 3, PMOsC2; lane 4, PMOsC3; lane 5, PMOsC4).

As can be seen in FIG. 4, as a result, α1,3-Fucose and β1,2-Xylose were hardly detected in the selected cell line. Through these results, it was confirmed that mutations of α1,3-FucT and β1,2-XylT enzyme genes occurred normally. Furthermore, the changes in the N-carbohydrate structure of the selected cell lines were analyzed in more detail.

To this end, the extracted protein was trypsinized, and then, PNGase A was treated to separate N-carbohydrates from the protein, which was analyzed using MALDI-TOF MS (matrix-assisted laser desorption ionization time-of-flight mass spectrometry).

**[Table 4]**

| Relative proportion of N-carbohydrate structure in total protein of PMOsC cell lines | | | | | |
|---|---|---|---|---|---|
| Carbohydrate structure | WT_cell | PMOsC1_cell | PMOsC2_cell | PMOsC3_cell | PMOsC4_cell |
| MM | 0.0 | 0 | 0 | 50.8 | 42.3 |
| MMX | 18.0 | 0 | 0 | 0 | 0 |
| MMXF | 55.8 | 0 | 0 | 0 | 0 |
| MGn | 0.0 | 19.2 | 7.7 | 13.8 | 26.4 |
| MGnX | 0.6 | 0 | 0 | 0 | 0 |
| MGnXF | 7.5 | 0 | 0 | 0 | 0 |
| GnGn | 0.0 | 80.8 | 92.3 | 35.4 | 31.3 |
| GnGnX | 0.0 | 0 | 0 | 0 | 0 |
| GnGnXF | 3.3 | 0 | 0 | 0 | 0 |
| AGnX | 0.1 | 0 | 0 | 0 | 0 |
| AGnF | 0.8 | 0 | 0 | 0 | 0 |
| AGnXF | 4.1 | 0 | 0 | 0 | 0 |
| AGnGnXF | 2.4 | 0 | 0 | 0 | 0 |
| AFGnGnXF | 3.8 | 0 | 0 | 0 | 0 |
| AAGnGnXF | 1.6 | 0 | 0 | 0 | 0 |
| AAFFGnGnXF | 1.9 | 0 | 0 | 0 | 0 |
| Total | 100 | 100 | 100 | 100 | 100 |

As can be seen in Table 4, although various N-carbohydrate structures were detected in WT, in the case of PMOsC1 and PMOsC2 cell lines, two types of N-carbohydrate structures, MGn and GnGn, were detected, and in the case of PMOsC3 and PMOsC4, three types of N-carbohydrate structures, MM, MGn and GnGn, were detected. In particular, the proportions of GnGn structures in PMOsC1 and PMOsC2 cell lines were 80.8% and 92.3%, respectively. On the other hand, in the case of PMOsC3 and PMOsC4 single cell lines, MM structures were detected the most, and the relative amounts thereof were 50.8% and 42.3%, respectively.

The difference among these cell lines is thought to be due to the mutation of the hexosaminidase (HEXA) gene corresponding to Hexo1 to Hexo4.

**[Table 5]**

| Relative ratio of N-carbohydrate structure of secreted protein secreted into medium of PMOsC1 cell line | | |
|---|---|---|
| Carbohydrate structure | WT_media | PMOsC1_media |
| MM | 0.0 | 1.0 |
| MMX | 2.7 | 0.0 |
| MMXF | 81.6 | 0.0 |
| MGn | 0.0 | 1.6 |
| MGnXF | 11.4 | 0.0 |
| GnGn | 0.0 | 97.4 |
| GnGnXF | 1.3 | 0.0 |
| AGnXF | 2.1 | 0.0 |
| AGnGnXF | 0.8 | 0.0 |
| Total | 100.0 | 100.0 |

As can be seen in Table 5, from the result of the secreted proteins secreted into the suspension culture medium of the PMOsC1 cell line, it can be seen that the MM, MGn, and GnGn structures showed ratios of 1.0%, 1.6%, and 97.4%, respectively, and the GnGn structure was more dominant than that in cells.

Through these results, mutations in 6 genes that showed nearly 100% gene editing efficiency in PMOsC1 and PMOsC2 were reconfirmed, and β1,3-GalT and α1,4-FucT genes, which showed relatively low gene editing efficiency, were found to have no detectable function in protein N-glycosylation.

Inhibition of the β1,3-GalT and α1,4-FucT enzymes in these selected cell lines is probably due to abnormal N-glycosylation caused by mutations in α1,3-FucT and β1,2-XylT that act prior to these enzymes, andthe production of abnormal matrix proteins lacking normal N-glycosylation modified with α1,3-fucose and β1,2-xylose, which are substrates for β1,3-GalT and α1,4-FucT enzymes.

In addition, the result of detecting very low amounts of MM structures or no detection indicates that the hexosaminidase genes have been mutated.

From the above results, we finally obtained PMOsC1 and PMOsC2 single cell lines which caused mutations in 8 genes, and from their N-glycosylation structural analysis, we were able to establish the cell lines that inhibited the function of 8 target enzymes. In addition, it was confirmed that 4 genes were successfully mutated through analysis of carbohydrate structures in PMOsC3 and PMOsC4 cell lines.

These cell lines are expected to be a useful resource for establishing an efficient cell line system and studying the function of the gene in order to conduct experiments related to environmental stimuli and stress in the future. In addition, these plant-specific glycosylation gene mutant cell lines can be used as a production platform capable of producing various useful proteins.

### Example 6: Expression of TMab, a breast cancer therapeutic agent, in plant-specific glycosylation mutant cell lines

Expression of breast cancer therapeutic agent TMab was attempted in plant-specific glycosylation gene mutant cell lines. The signal peptide sequence (GKHHVTLCCWFAVLCLASSLAQA) of the Amylase 3E (RAmy3E) protein of rice was added to the 5' ends of the light and heavy chains of TMab, and then, the genes of TMab light and heavy chains, which were codon-optimized to rice codons, were synthesized (GeneArt, Germany) and introduced into the pEAQ-HT vector (Sainsbury et al., 2009) to construct pSK446 vector. Such an expression vector was used to transform PMOsC1 cell line, and then, selected in 50 mg/L of G418 (Geneticin). As a control, proteins extracted from untransformed WT (Dongjin) callus lines were used.

**[Table 6]**

| SEQ ID NO. | Name | Sequence |
|---|---|---|
| 41 | TMab light chain amino acid sequence | DIQMTQSPSSLSASVGDRVTITCRASQDVNTAVAWYQQKPGKAPKLLIYSASFLYSGVPSRFSGS RSGTDFTLTISSLQPEDFATYYCQQHYTTPPTFGQGTKVEIKRTVAAPSVFIFPPSDEQLKSGTAS VVCLLNNFYPREAKVQWKVDNALQSGNSQESVTEQDSKDSTYSLSSTLTLSKADYEKHKVYACE VTHQGLSSPVTKSFNRGEC |
| 42 | TMab heavy chain amino acid sequence | EVQLVESGGGLVQPGGSLRLSCAASGFNIKDTYIHWVRQAPGKGLEWVARIYPTNGYTRYADSV KGRFTISADTSKNTAYLQMNSLRAEDTAVYYCSRWGGDGFYAMDYWGQGTLVTVSSASTKGPS VFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGPSVFLFPPKPKDTLMI SRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEW ESNGQPENNYKTTPPVLDSDGSFFLYSKLTVDKSRWQQGNVFSCSVMHEALHNHYTQKSLSLS PGK |
| 43 | RAmy3E signal peptide amino acid sequence | GKHHVTLCCVVFAVLCLASSLAQA |
| 44 | TMab light chain synthesis gene (including RAmy3E signal peptide sequence) | ATGGGCAAGCACCACGTGACCCTGTGCTGCGTGGTGTTTGCTGTTCTGTGCCTGGCAAGCA GCCTGGCACAGGCAGACATCCAGATGACACAGAGCCCTAGCAGCCTGAGCGCATCTGTGG GCGATCGCGTGACAATTACATGCCGCGCAAGCCAGGACGTGAACACCGCAGTTGCATGGTA CCAGCAGAAGCCAGGCAAGGCCCCAAAGCTGCTGATCTACAGCGCCAGCTTCCTGTACAGC GGCGTGCCATCTCGCTTTAGCGGTTCTCGCAGCGGCACCGATTTCACCCTGACAATCTCTAG CCTGCAGCCAGAGGACTTCGCCACCTACTACTGCCAGCAGCACTACACCACACCACCAACA TTCGGCCAGGGCACCAAGGTCGAGATCAAGCGAACAGTGGCAGCCCCAAGCGTGTTCATCT TCCCACCATCTGACGAGCAGCTGAAGTCCGGCACAGCTTCTGTGGTGTGCCTGCTGAACAA CTTCTACCCACGCGAGGCCAAGGTGCAGTGGAAGGTTGACAACGCACTGCAGAGCGGCAA CAGCCAGGAGAGCGTTACAGAGCAGGACAGCAAGGACTCCACCTACAGCCTGTCCAGCACA CTGACCCTGAGCAAGGCCGACTACGAGAAGCACAAGGTGTACGCCTGCGAGGTGACACAC CAGGGCCTGTCATCTCCAGTGACCAAGTCTTTCAACCGCGGCGAGTGCTGATGA |
| 45 | TMab heavy chain synthesis gene (including RAmy3E signal peptide sequence) | ATGGGCAAGCACCACGTGACCCTGTGCTGCGTGGTGTTTGCTGTTCTGTGCCTGGCAAGCA GCCTGGCACAGGCAGAGGTTCAGCTGGTTGAGTCTGGCGGTGGTCTGGTTCAGCCTGGTG GTTCTCTGCGACTGTCTTGCGCAGCAAGCGGCTTCAACATCAAGGACACCTACATCCACTGG GTGCGCCAGGCACCAGGTAAGGGCCTGGAGTGGGTTGCACGCATCTACCCTACCAACGGC TACACCCGCTACGCCGATTCTGTGAAGGGCCGCTTTACCATCAGCGCCGACACCAGCAAGA ACACCGCCTACCTGCAGATGAACAGCCTGCGCGCTGAGGACACCGCAGTGTACTACTGCTC TCGATGGGGCGGTGACGGCTTCTACGCAATGGATTACTGGGGCCAGGGCACCCTGGTGAC AGTGTCTAGCGCATCTACAAAGGGCCCAAGCGTGTTCCCACTGGCACCAAGCAGCAAGTCT ACAAGCGGCGGTACAGCAGCACTGGGCTGCCTGGTGAAGGACTACTTTCCAGAGCCAGTGA |
| | | CCGTGAGCTGGAACAGCGGTGCACTGACATCTGGCGTGCACACATTCCCAGCAGTGCTGCA GTCAAGCGGCCTGTACTCTCTGAGCAGCGTGGTGACCGTGCCATCTTCTTCACTGGGCACC CAGACCTACATCTGCAACGTGAACCACAAGCCAAGCAACACCAAGGTGGACAAGAAGGTCG AGCCAAAGAGCTGCGACAAGACCCACACATGCCCACCATGCCCTGCACCAGAGCTGCTCGG TGGCCCATCTGTGTTTCTGTTCCCACCAAAGCCAAAGGACACCCTGATGATCAGCCGCACAC CAGAGGTGACATGCGTGGTGGTGGATGTGAGCCACGAGGACCCTGAGGTGAAGTTCAACTG GTACGTGGACGGCGTCGAGGTGCACAACGCAAAGACAAAGCCACGCGAGGAGCAGTACAA CAGCACCTACCGAGTGGTGTCCGTGCTCACTGTGCTGCACCAGGATTGGCTGAACGGCAAG GAGTACAAGTGCAAGGTGAGCAACAAGGCCCTGCCAGCACCAATCGAGAAGACCATCAGCA AGGCCAAGGGCCAGCCACGAGAGCCACAGGTTTACACCCTGCCACCATCTCGCGAGGAGA TGACCAAGAACCAGGTGAGCCTGACCTGCCTCGTCAAGGGCTTCTACCCAAGCGACATTGC CGTCGAGTGGGAGTCTAACGGCCAGCCTGAGAACAACTACAAGACCACACCACCAGTGCTG GACAGCGACGGCTCATTCTTCCTGTACAGCAAGCTGACCGTGGACAAGTCCCGATGGCAGC AGGGCAACGTGTTCAGCTGCTCTGTGATGCACGAGGCCCTGCACAACCACTACACCCAGAA GTCCCTGAGCCTGTCTCCAGGCAAGTGATGA |

The selected calli were cultured in suspension for 2 weeks, and the culture medium was recovered therefrom and after centrifugation at 440 g x 5 minutes, concentration was performed with Vivaspin (50 MWCO, Sartorius). The concentrated solution was subjected to immunoblotting using antibodies specific for human IgG gamma chain and kappa chain (AP309P, AP502, PMillipore, USA) (1, size marker; 2, concentrate of medium used for suspension culture of TMab transformed cell line; 3, WT (negative control) 4, Herzuma (analogue of TMab, positive control).

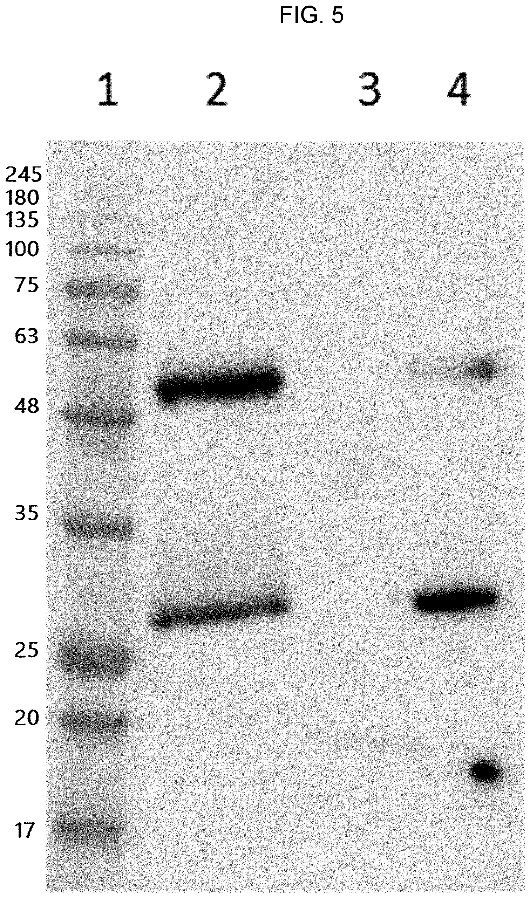

As can be seen in FIG. 5, it was confirmed that both the light chain and the heavy chain were normally produced in the cell line.

### Industrial Applicability

The present disclosure relates to N-glycosylation mutant rice (*Oryza sativa*), a method of producing the same, and a method of producing rice for protein production using the same, and more particularly, to a technique for establishing a rice cell line capable of producing medical proteins by editing a total of eight genes which participate in a plant-specific N-glycosylation process, to remove plant-specific glycosylation.

## Claims

1. A vector for editing a rice (*Oryza sativa*) N-glycosylation gene comprising a guide RNA consisting of one or more nucleotide sequences selected from the group consisting of SEQ ID NOS: 1 to 16.

2. The vector of claim 1, wherein the guide RNA targets one or more genes selected from the group consisting of β1,2-XylT, α1,3-FucT, β1,3-GalT, α1,4-FucT, HEXO1, HEXO2, HEXO3, and HEXO4.

3. The vector of claim 1, wherein the guide RNA is a polycistronic guide RNA designed to express two or more nucleotide sequences selected from the group consisting of SEQ ID NOS: 1 to 16.

4. An N-glycosylation mutant rice (*Oryza sativa*) transformed with a vector comprising a guide RNA consisting of one or more nucleotide sequences selected from the group consisting of SEQ ID NOS: 1 to 16.

5. A composition for producing Trastzumab (TMab), which is codon-optimized so that TMab is expressed in rice (*Oryza sativa*) and includes a TMab light chain synthetic gene consisting of the nucleotide sequence of SEQ ID NO: 44 and a TMab heavy chain synthetic gene consisting of the nucleotide sequence of SEQ ID NO: 45.

6. A vector for expressing trastuzumab (TMab), which is codon-optimized so that TMab is expressed in rice (*Oryza sativa*) and includes a TMab light chain synthetic gene consisting of the nucleotide sequence of SEQ ID NO: 44 and a TMab heavy chain synthetic gene consisting of the nucleotide sequence of SEQ ID NO: 45.

7. A rice for producing Trastuzumab (TMab), the rice transformed with:
a vector for editing a rice (Oryza sativa) N-glycosylation gene, the vector comprising a guide RNA consisting of one or more nucleotide sequences selected from the group consisting of SEQ ID NOS: 1 to 16; and
a vector for expressing TMab, which is codon-optimized so that TMab is expressed in rice and includes a TMab light chain synthetic gene consisting of the nucleotide sequence of SEQ ID NO: 44 and a TMab heavy chain synthetic gene consisting of the nucleotide sequence of SEQ ID NO: 45.

8. The rice of claim 7, wherein the guide RNA targets one or more genes selected from the group consisting of β1,2-XylT, α1,3-FucT, β1,3-GalT, α1,4-FucT, HEXO1, HEXO2, HEXO3, and HEXO4.

9. The rice of claim 7, wherein the guide RNA is a polycistronic guide RNA designed to express two or more nucleotide sequences selected from the group consisting of SEQ ID NOS: 1 to 16.
